# EUROPEAN PATENT SPECIFICATION

(11) **EP 2 720 798 B1**
(45) Date of publication and mention of the grant of the patent: **01.01.2020**
(21) Application number: 12732711.2
(22) Date of filing: 14.06.2012
(51) Int. Cl.: B03C 1/01, B03C 1/28, G01N 33/543

(54) **PROCESSING OF BIOLOGICAL SAMPLE COMPONENTS**
VERARBEITUNG DER KOMPONENTEN BIOLOGISCHER PROBEN
TRAITEMENT DE COMPOSANTS D'ÉCHANTILLON BIOLOGIQUE

(30) Priority: 15.06.2011 EP 11169981
(43) Date of publication of application: 23.04.2014
(73) Proprietor: Koninklijke Philips N.V., 5656 AE Eindhoven (NL)
(72) Inventor: OVSYANKO, Mikhail, Mikhaylovich, NL-5656 AE Eindhoven (NL); VAN HEMERT, Freek, NL-5656 AE Eindhoven (NL); PRINS, Menno, Willem, Jose, NL-5656 AE Eindhoven (NL); VAN DE STOLPE, Anja, NL-5656 AE Eindhoven (NL); WIMBERGER-FRIEDL, Reinhold, NL-5656 AE Eindhoven (NL)
(74) Representative: Philips Intellectual Property & Standards
(86) International application number: PCT/IB2012/053005
(87) International publication number: WO 2012/172503

(56) References cited:
- EP-A1- 1 936 350
- EP-B1- 1 144 073
- WO-A1-2009/060357
- WO-A1-2010/049883
- WO-A1-2010/109392
- WO-A1-2010/121315
- BRULS D M ET AL: "Rapid integrated biosensor for multiplexed immunoassays based on actuated magnetic nanoparticles", LAB ON A CHIP, ROYAL SOCIETY OF CHEMISTRY, vol. 9, no. 24, 15 October 2009 (2009-10-15), pages 3504-3510, XP002570598, ISSN: 1473-0197, DOI: 10.1039/B913960E [retrieved on 2009-10-15]

## Description

### FIELD OF THE INVENTION

The invention relates to the processing of a biological sample fluid comprising different components, particularly comprising cells labeled with magnetic particles.

### BACKGROUND OF THE INVENTION

In a variety of biological assays, specific components of a sample shall be prepared for optical examination, wherein these components first have to be enriched and/or separated from other parts of the sample. The analysis of rare cells requires for example technologies to enrich or isolate these cells before they can be further analyzed in detail.

EP 1 144 073 B1 discloses apparatuses and methods for separating, immobilizing, and quantifying biological substances from within a fluid medium. Biological substances are observed by employing a vessel having a chamber therein, the vessel comprising a transparent collection wall. A high internal gradient magnetic capture structure may be on the transparent collection wall, wherein magnets create an externally-applied force for transporting magnetically responsive material toward the transparent collection wall.

WO 2009/060357 A1 discloses a cartridge comprising a sample input portion and a sensor portion, wherein said sensor portion comprises a sensor surface and a first microstructure adapted to provide a capillary force for transporting sample fluid from the sample input portion to the sensor portion, wherein said microstructure does not interfere with the sensor surface.

### SUMMARY OF THE INVENTION

It is an object of the invention to provide means that allow for a simple and reliable processing of biological samples comprising components of interest that shall be optically treated or examined.

This object is achieved by a method according to claim 1 and a processing device according to claim 2. Preferred embodiments are disclosed in the dependent claims.

A method according to the invention relates to the processing of a sample fluid comprising different components, particularly a sample fluid of biological origin like blood or saliva. The method comprises the following steps:
a) Introducing the sample fluid into a first compartment of a container. This introduction may take place passively, for example with the help of capillary forces by which the sample fluid is driven into the first compartment, or it may actively be assisted by hydrodynamic forces or the like. It should be noted that the term "compartment" shall, in the context of the present invention, refer to a space of nearly arbitrary geometry that may be filled by a fluid. A compartment may particularly be comprised of one or more chambers (i.e. open cavities having a compact - e.g. cuboid - geometry) connected by channels.
b) Letting in a first phase at least one selected component of the sample pass through a filtering element that separates the first compartment from a second compartment while the passage of at least one other component of the sample is blocked. The passage of sample components through the filtering element may take place passively, i.e. only driven by diffusion, or it may be actively assisted, for example by applying a hydrodynamic pressure or a non-homogeneous magnetic field.
c) Collecting the at least one other component of the sample on an optical surface that is disposed in the first compartment or collecting the at least one selected component on an optical surface that is disposed in the second compartment of the container. Again, this collection may take place passively or may actively be assisted. The optical surface may be disposed in the first compartment (for example in any or all of its chambers) if the selected component that passes the filtering element is of no interest with respect to optical processing. If the selected component is of interest, the optical surface will typically be disposed in the second compartment. Of course it is also possible to collect (different) components of the sample on two optical surfaces disposed in the first and the second compartment, respectively. The optical surface may be any surface on which optical processes of a desired kind may take place, for example a surface on which the components can optically be imaged by a microscope, a camera or the like.
d) Generating during the first phase a magnetic field with a nonzero field gradient across the filtering element and with field lines that are perpendicular to the filtering element.
e) Generating after the first phase a magnetic field with field lines that are parallel to the filtering element.

The components of the sample fluid may preferably be components labeled with magnetic particles, wherein the term "magnetic particles" shall comprise both permanently magnetic particles as well as magnetizable particles, for example superparamagnetic beads. The size of the magnetic particles typically ranges between 3 nm and 50 µm.

The invention further relates to a processing device for processing a sample fluid containing different components. The processing device comprises the following components:
- A container with a first compartment and a second compartment, both of which may optionally be comprised of multiple chambers connected by channels, wherein the first compartment can be filled with the sample fluid.
- A filtering element that is disposed between said first and second compartments and that allows in a first phase the passage of at least one selected component of the sample while it blocks the passage of at least one other component.
- An optical surface that is disposed in the first compartment and on which the at least one other component of the sample can collect or that is disposed in the second compartment and on which the at least one selected component of the sample can collect.
- A first magnet arranged to generate during the first phase a magnetic field with a nonzero field gradient across the filtering element and wit field lines running substantially perpendicular to said filtering element.
- A further magnet arranged to generate after the first phase a magnetic field that has field lines running substantially in parallel with said filtering element.

With the processing device, a method of the kind described above can be executed. Explanations and definitions provided for the method are therefore also valid for the processing device and vice versa. The method and the processing device allow for a simple and reliable processing of a biological sample, for example a preparation for optical treatments. This is achieved by the provision of a container with a filtering element by which different components of the sample can selectively be separated into two compartments. This allows for collection of components of interest faster, with higher concentration, and with a higher degree of purity on an optical surface, which in turn increases the accuracy and efficiency of subsequent optical processing steps.

In the following, various preferred embodiments of the invention will be disclosed that relate to both a method and a processing device of the kind described above.

According to a first preferred embodiment, the filtering element comprises a structure that is capable of leaving objects below a certain size through while blocking large ones. Such a structure may particularly comprise pores with a diameter smaller than a given part of the sample (the target). The target will therefore not be able to pass through the filtering element, yielding a separation of the sample components with respect to their size. The appropriate threshold of the maximal diameter will be chosen in dependence on the composition of the sample fluid at hand, with typical values ranging between about between 0.05 µm and 50 µm.

According to another aspect of the invention, the filtering element can specifically bind at least one component of the sample and/or specifically repel at least one component of the sample. By binding a component of the sample, the filtering element effectively removes this component so that it can no longer collect on the optical surface. Coating the filtering element for example with anti-CD45 allows to reduce leukocyte background of a rare cell sample. By repelling a component of the sample, the filtering element prevents this component from passing from the first into the second compartment. Both binding and repulsion may be very specific with respect to the components they act on. Moreover, these processes allow for a separation that is based on other characteristics than particle size, for example a separation based on electrical charge, chemical composition or the like.

The processing device or the method may further comprise the ability to produce a hydrodynamic pressure gradient across the filter element. A pressure generator may for example be provided for generating a hydrodynamic pressure across the filtering element (e.g. a pressure that is higher in the first compartment than in the second compartment). Such a hydrodynamic pressure will assist the flow of sample fluid through the filtering element and thus accelerates the filtering process. The pressure generator may comprise both means for generating an overpressure as well as means for generating an underpressure with respect to the "normal" pressure within the sample fluid.

According to another embodiment of the invention, rotation means may be provided for generating centrifugal forces across the filtering element that may accelerate the filtering process. The rotation means may for example comprise an apparatus in which one or more containers according to the invention can be accommodated and (commonly) be rotated.

The first magnet and/or the further magnet may particularly comprise a permanent magnet and/or an electromagnet. With the help of a magnetic field and particularly a field gradient, magnetic particles within the sample can be actuated and for instance be moved in a desired direction, thus accelerating migration processes within the sample. Moreover, they can be used to fix (immobilize) components having a high (induced) magnetic moment on any of the container's surfaces while components with smaller or zero magnetic moments are washed away.

The aforementioned first magnet and/or further magnet may particularly be adapted to generate a magnetic field with field lines that are perpendicular and/or with field lines that are parallel to the filtering element. While the direction of a field gradient determines the direction of magnetic forces acting on magnetic particles in the sample, the direction of the field lines allows for affecting the direction to which clusters or strings of magnetic particles (or rotationally asymmetric magnetic particles) align.

As mentioned above, a magnetic field with a nonzero gradient across the filtering element and with field lines (approximately) perpendicular to the filtering element is generated during a first phase after introduction of the sample into the first compartment of the container. Due to the field gradient, magnetic particles within the sample will be exposed to a force pulling them across the filtering element, whereby strings of such particles will be oriented perpendicular to the filtering element in alignment to the field lines. As a result, complete strings of magnetic particles can pass the filtering element (if its pores are large enough to allow the passage of single magnetic particles).

As further mentioned above, a magnetic field with field lines parallel to the filtering element is generated after the first phase. In the region of the filtering element, this field will cause strings of magnetic particles to be oriented parallel to the filtering element, which will prevent them from passing through the pores of the filter (provided that the pores are smaller than the length of the strings). Magnetic particles that have passed the filtering element in the first phase, i.e. when the field lines were perpendicular to the filter, will therefore be retained in the chamber they have reached during this subsequent phase.

In another embodiment of the invention, the optical surface is detachable from the container, particularly in such a way that it can be easily reattached without alterations to the device as a whole for subsequent sample processing. This allows for usage of the container for the processing of the sample, resulting in a collection of components of interest on the optical surface, which can then be transferred to another place for further processing.

Depending on the intended optical treatment or examination, the optical surface may have quite different, specific designs. In a preferred embodiment, the optical surface is provided by a (standard) microscopy slide, which is preferably arranged removable in one chamber of the compartments, forming for example a wall of the chamber. This allows for usage of the optical surface in many different optical examination apparatus.

A microscope objective or other features required for optical analysis such as a single lens or one of more optical fibers may be arranged at the container of the processing device in such a way that it allows a direct observation of the optical surface. Preparation of sample components and optical evaluation can then be executed in one and the same device.

The optical surface may optionally comprise binding sites that specifically bind at least one component of the sample. Thus this component can specifically be immobilized for a later processing.

The first and/or the second compartment may be provided with at least one fluidic connection, for example an inlet and an outlet, such that a fluid flow through this compartment can be generated. Thus fresh sample fluid can for example continuously or intermittently be supplied to the first compartment. In the second compartment, components of the sample that have traversed the filtering element can be removed with a flow, thus preventing their return into the first compartment.

It was already said that the compartments may comprise one or more chambers. In a preferred embodiment, the first compartment and/or the second compartment comprises a single chamber, having on one side the filtering element and on another side the optical surface.

### BRIEF DESCRIPTION OF THE DRAWINGS

These and other aspects of the invention will be apparent from and elucidated with reference to the embodiments described hereinafter.

In the drawings:
- Fig. 1: shows a schematic side view of a processing device according to a first embodiment of the invention, wherein both the first and the second compartment have an inlet and an outlet;
- Fig. 2-5: show consecutive steps of a method according to the invention;
- Fig. 6 and 7: show consecutive steps of a method according to the invention, wherein magnetic field lines perpendicular and parallel to the filtering element are generated;
- Fig. 8: shows a processing device with an integrated microscope objective;
- Fig. 9: shows an example of a processing device where the first compartment is split in adjoining chambers, connected by a channel;
- Fig. 10: shows an example of a processing device where the first compartment is split in adjoining chambers, connected by a channel that comprises a valve construction.

Like reference numbers or numbers differing by integer multiples of 100 refer in the Figures to identical or similar components.

### DETAILED DESCRIPTION OF EMBODIMENTS

In the following, the invention will be described with respect to the processing of rare cells, though it can be applied in a variety of other areas, too. Rare cells, such as circulating tumor cells (CTCs), can be effectively enriched from large pools of other cells by means of immuno-magnetic capture. This method entails antibodies (against specific epitopes) covalently coupled to superparamagnetic beads. The coupling of such beads to cells provides a way to specifically exert a force on the cells that express these specific epitopes. By magnetically locking the labeled cells in position while washing other cells away, very high levels of enrichment can be achieved. To enable standard pathology diagnostic staining protocols to be applied to CTCs, the CTCs need to be presented on a standard microscopy slide.

For an efficient, immuno-magnetic enrichment of rare cells a large excess of immuno-magnetic particles (beads) needs to be added to the sample compared to the space available on the surface of the to be selected cells. Due to their magnetic nature this excess of beads will stay with the cells throughout the following enrichment protocol. Thus the final sample will, next to the enriched cells, also contain a large quantity of free immuno-magnetic beads. These beads will deteriorate the quality of fluorescent images taken of the cells. Moreover, during normal microscopy, it is hard to judge cell morphology in the presence of large bead clusters. It is therefore desirable to have a device that resolves these issues by effectively removing all the free immuno-magnetic beads while not altering the cell/bead clusters.

A processing device according to an embodiment of the present invention addresses the above issues and combines functionalities for removing (excess) immuno-magnetic beads or any other smaller particles or cells (like erythrocytes) from suspensions of rare cells. The rare cells are subsequently and as an integral part of the assay deposited on an optical surface suited for optical imaging, e.g. in a microscope for cell imaging. In general, the processing device is characterized by the following properties:
- It has a first compartment, this compartment may be comprised of one or more chambers connected by one or more channels.
- At least one of the chambers of the first compartment is bordered on one side by a filtering element with pores.
- At least one of the chambers of the first compartment is bordered on one side by an optical imaging surface.

The processing device can be used according to the following method:
- A sample is introduced into the first compartment.
- Small sample components pass through the filtering element, for example by applying hydrodynamic, centrifugal or magnetic forces.
- Large sample components are actuated through the first compartment (possibly to another chamber) toward the optical surface, for example by magnetic forces, gravitational forces and/or hydrodynamic forces.
- The large sample components are optically imaged on the optical surface.

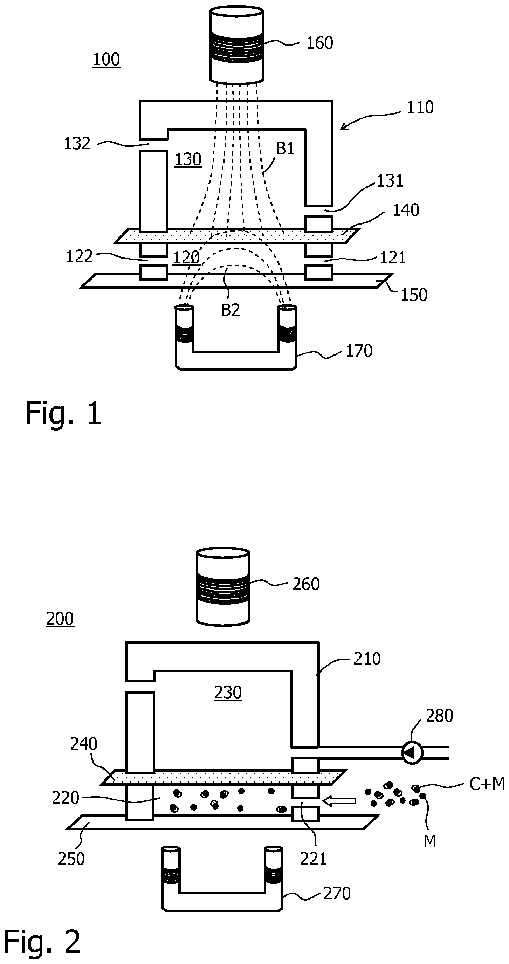

Figure 1 schematically shows a side view of a processing device 100 according to a first embodiment of the present invention. The processing device 100 comprises a container 110 with a first compartment 120 and a second compartment 130. The depicted container may be radially symmetric but could also be rectangular. It is able to attach to and seal a standard microscopy slide 150 at the bottom of the first compartment 120 using a vacuum connection, by clamping or by gluing. The microscopy slide 150 provides an optical surface facing the interior of the first compartment 120. The first and the second compartments are separated by a filtering element 140 of any suited material.

Moreover, the processing device 100 comprises a first magnet 160 arranged close to (above) the second compartment 130, and a second magnet 170 arranged close to (below) the first compartment 120. As indicated by dotted lines, the first magnet 160 can generate a magnetic field B1 with field lines substantially perpendicular to the filtering element 140, while the second magnet 170 can generate a magnetic field B2 with field lines substantially parallel to the filtering element. Both magnets 160 and 170 generate magnetic fields with a nonzero field gradient across the filtering element 140 (wherein the gradient of the first magnet 160 is directed from the first to the second compartment, while the gradient of the second magnet 170 is oppositely directed). The lower magnet 170 is preferably designed in such a way that the magnetic field is uniform over the cell deposition area, facilitating an even distribution of cells over the microscopy slide surface.

The first compartment 120 of the processing device 100 allows for the injection of a sample through an inlet 121, the sample typically being a mixture of cells, immuno-magnetic beads and complexes of these two. The presence of an outlet 122 allows for flow through the first compartment 120, for example to allow for fixatives or other reagents (antibodies, FISH probes, etc) to be flushed in and out of the compartment, possibly after removal of excess immuno-magnetic beads. The labeled cells may be kept in place during these procedures using the lower magnet 170.

The second compartment 130 may hold a buffered aqueous solution. In the embodiment of Figure 1 this compartment also features an inlet 131 and an outlet 132 allowing for flow.

The solenoid electromagnet 160 at the top of the second compartment 130 can exert forces on (superpara-)magnetic particles present in the first compartment 120 and move them to the second compartment 130 across the filtering element 140 with variable pore size and surface. In case the beads are connected to cells or sufficiently large, the magnet pulls them against the filter 140. When small cells are labeled with beads they will also move through the filter, depending on pore size, realizing an extra selection step in the protocol.

The large sample components that cannot pass the filtering element 140 can be cells, aggregates of cells, or larger pieces of tissue that are suspended in the sample fluid. They can be coupled onto the optical surface 150 (e.g. for robust further processing of the cells) or can remain unattached (e.g. to minimize cell activation or to allow further cell manipulation and transportation). The coupling of cells to the optical surface can be mediated by a dedicated surface layer (e.g. poly-L-lysine), can be chemically enhanced (e.g. by a protein fixative), or can be done by applying a covering matrix (e.g. a paraffin layer). The usage of a standard microscopy slide 150 leaves the cells accessible to any standard microscope or any custom design staining/fixing procedure.

By using a filtering element 140 having a porous surface with a defined pore size, free immuno-magnetic beads and/or other particles may be selectively removed from the sample. By using a solenoid magnet, resulting in magnetic field lines perpendicular to the filter, beads will easily move from the first compartment to the second compartment. A horseshoe magnet at the base of the device will generate a magnetic field with field lines parallel to the filter, preventing the beads from reentering the first compartment while fixing the cells on the cover glass. Alternatively, with some minor alterations to the device, a pressure difference may be used as the driving force across the size selection filter. Using a vacuum connection to fix a standard microscopy slide to the device will make the device easy to use.

While the described processing device 100 is able to pull cells towards a standard microscope glass slide using a magnet 170, gravity could alternatively be used combined with specific glass coatings to facilitate cell binding.

Figures 2 to 5 illustrate consecutive steps of an exemplary cell preparation. The processing device 200 used for this purpose is s slightly modified version of the device 100 described above.

Figure 2 illustrates the first step, in which a sample containing immuno-magnetically labeled cells, C+M, and an excess of immuno-magnetic beads, M, is loaded into the first compartment 220 of a container 210 through an inlet 221.

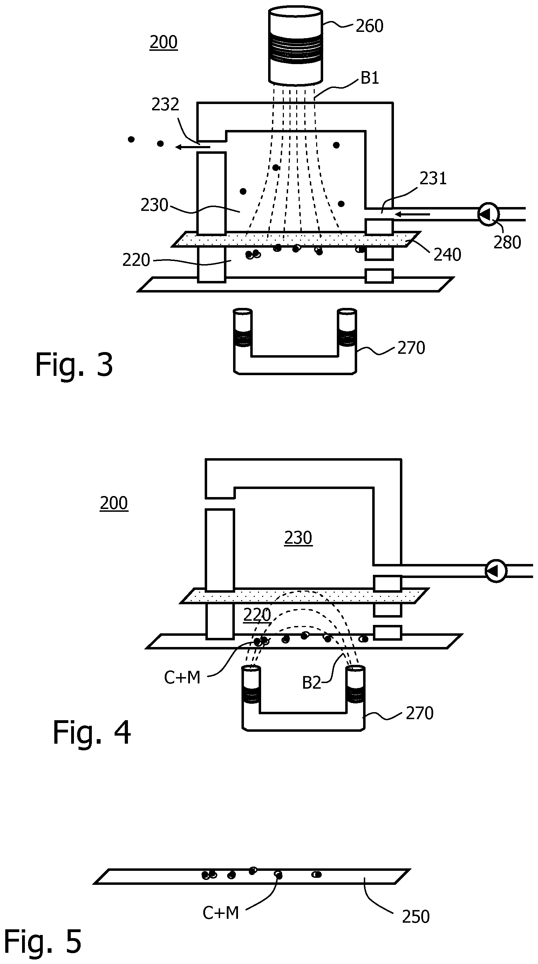

According to Figure 3, the magnetic beads M are then pulled through the filtering element 240 with the help of the upper magnet 260 while the labeled cells C+M remain in the sample loading area 220. Applying flow through second compartment 230, the beads M can be removed from the second compartment. The flow can be generated with a pump 280.

Figure 4 shows the activation of the lower magnet 270, generating a magnetic field that pulls labeled cells C+M from the filter 240 onto the optical surface 250 (microscopy slide). After having removed all liquid from inside the container, especially from the first compartment 220, the entire container 210 can be removed leaving the user with a microscopy slide 250 containing only magnetically labeled cells (Figure 5). The filter and microscopy slide are exchangeable and can optionally be functionalized.

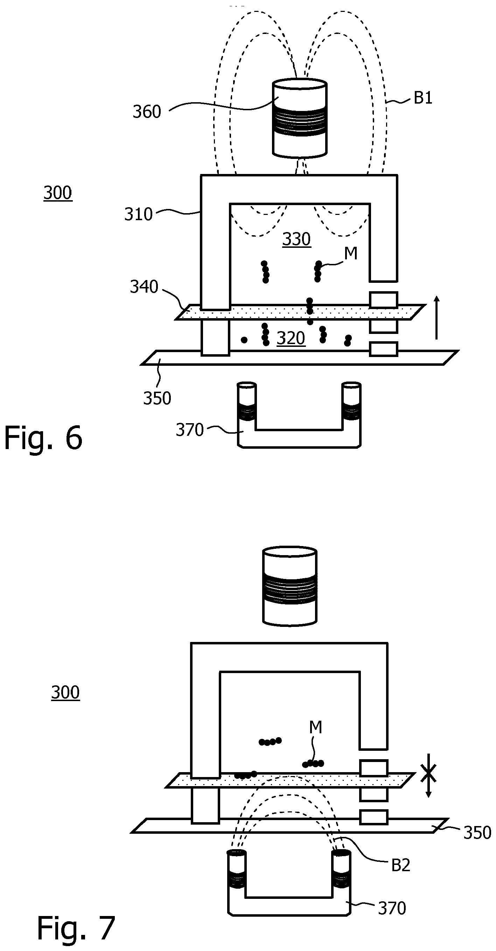

Figures 6 and 7 show an embodiment of the processing device 300 in which the second (upper) compartment 330 is closed for liquids (but vented) and magnetic trapping (i.e. bead strings become perpendicular to the filter surface) is used as the sole mechanism of separating beads M and labeled cells C+M. This will simplify the device architecture.

Figure 6 shows a first phase in a procedure of magnetic trapping after filling of the first compartment 320 with a sample. A (e.g. solenoid) magnet 360 creates magnetic field lines B1 that are (approximately) perpendicular to the filter 340. These filed lines facilitate the passage of strings of beads, which tend to align to the field lines.

According to Figure 7, a (e.g. horseshoe) magnet 370 below the first compartment 320 is subsequently used to pull the immuno-magnetically labeled cells C+M towards the microscope glass slide 350 and hold them there. A horseshoe magnet will produce a steeper field gradient and thus a higher force on the beads and bead/cell complexes. Simultaneously the field lines B2 orient the bead strings parallel to the filter 340 thereby preventing them from passing the filter and entering the first compartment again. This means that the second compartment 330 does not need connections for rinsing or washing because it is not necessary to wash away the immuno-magnetic beads in order to prevent them from reentering the sample area.

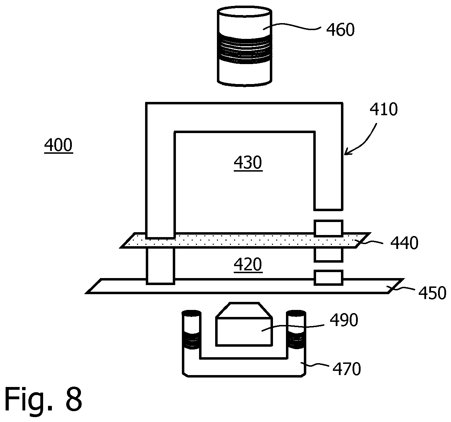

Figure 8 illustrates a processing device 400 that comprises an integrated microscope objective 490. Cells may then be visualized and counted while remaining in the device.

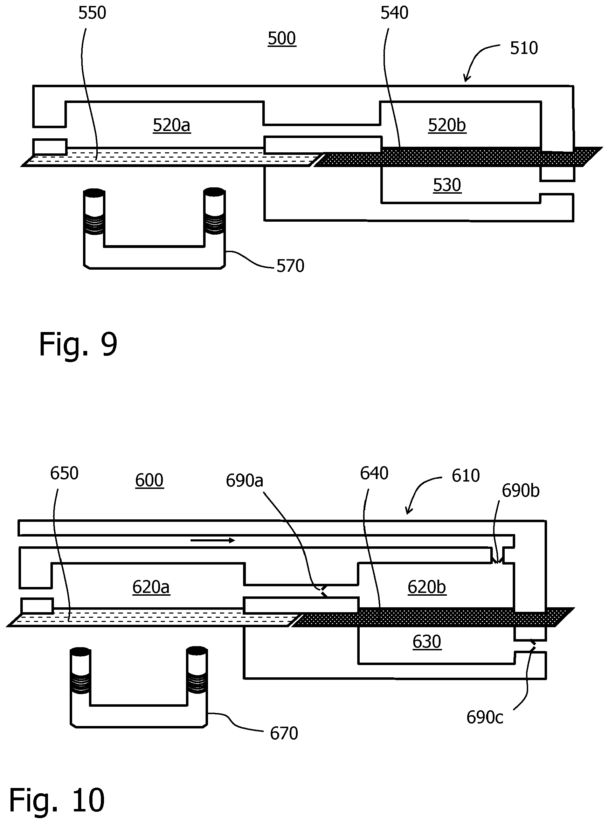

Figure 9 shows an example of a processing device 500 where the first compartment is split in adjoining chambers 520a and 520b, connected by a channel.

Figure 10 shows another example of a processing device 600 where the first compartment is split in adjoining chambers 620a and 620b, connected by a channel. A valve construction comprising valves 690a, 690b, 690c allows for filtering of the sample before inverting the fluid flow and pumping the sample towards the optical detection/analysis chamber 620a.

The described realizations of the invention may be modified or extended in many ways, some of which will be described below.

The microscopy slide may be attached in a reversible way by an adhering "sticky" interface between container and microscopy slide. The interface may comprise a sticky rubber layer or a glue e.g. double sided tape. In an alternative embodiment the microscopy slide is attached to the cartridge using vacuum pressure.

The processing device may optionally be rotated upside down in its entirety. This allows for reversal of the direction in which gravity drives particles across the filtering element. Such an approach will minimize the sheer stresses on cells.

In another embodiment the processing device is used to separate lesser magnetically labeled objects from cells that have bound a lot of magnetic beads by means of fixing well labeled cells in place while manipulating other objects by pressure difference induced flows. This may serve to increase the enrichment of CTCs by depleting aspecifically labeled leukocytes.

In one embodiment the filtering element (the porous material) is functionalized to prevent binding of cells and/or the microscopy slide (the optical surface) contains reagents to encourage cell adhesion. Alternatively, the filter may be functionalized with antibodies against cells that are aspecifically labeled, for example, the filter could be coated with anti-CD45 antibodies to bind Leukocytes to it. These Leukocytes will not be imaged in this way.

While a typical filtering element may comprise a porous membrane, the filtering element may also be a complex filter containing structures that will only allow cells that have a certain degree of floppiness to pass through combined with a certain size/volume. Typical examples of suited materials for filtering elements are organic or inorganic materials, e.g.:
polycarbonate
Nylon
Nitrocellulose
silicon
polydimethylsiloxane (PDMS).

In one embodiment multiple layers of filters are combined to increase the effectiveness and throughput of the filtering element.

The lower magnet may be altered (permanently or dynamically) in such a way that the resulting field will concentrate the magnetically labeled cells in one defined spot on the microscopy slide. By achieving higher cell densities scanning times are reduced and problems with auto-focusing microscopes (which require a minimum sample density) are avoided.

In one embodiment the first compartment may be smaller than shown in the Figures while the microscope slide can be moved laterally. This allows for the deposition of multiple samples on one microscope glass slide.

In a further embodiment different areas of the microscopy slide are functionalized with different reagents that allow for (partial) segregation of the cells in the target area. This will result in different patterning for different cellular species. Patterns could be constructed from antibodies or surface altering chemicals.

In one embodiment the processing device is used as a size selection device. Large quantities of body fluids can be pumped through the filtering element after which the cells with size and stiffness parameters that prevent them from crossing the filter may be easily transferred to the microscopy slide, e.g. by using the magnet or by (pulsed) pressure inversion and subsequent sedimentation towards the glass surface.

A modified embodiment of the processing device may also pressurize (or vacuum) the first and/or the second compartment in order to create an extra driving force across the filtering element. The magnets can then be used to manipulate labeled objects independently of the pressure driven flows. The pressure gradient over the filter can be adjusted to the required order of magnitude to be compatible with magnetic forces for the desired bead arrangement (strings, etc.)

In summary, the invention provides a processing device to enable transfer of cells, especially circulating tumor cells, in suspension to a glass microscopy slide, and removing unbound magnetic beads for an improved image quality. The slide can either be processed for fixation and staining steps, or removed to enter the routine pathology workflow. Modularity of the system enables a multitude of other (molecular) biological functionalities without significant alteration to the core device, among cell separation based on magnetic labeling or size and stiffness. The filter of the processing device may be changed, and the device can be washed by reversed flow and/or using washing buffers.

Further features of the invention are:
- A device with a filter, an optical substrate and a magnet for separating magnetic beads from a mixture of a biological sample and magnetic beads.
- Application of a horse-shoe magnet.
- Application of an additional magnet opposite to the first magnet.
- Optical substrate being removable from the device.
- The filter being a 3D structured filter.
- The optical substrate having a layer for the specific binding of cells or for suppressing this binding.
- Allows for high volume microfluidics/fluid pumping.
- Staining procedures can be carried out after fixation.
- Microscopy slide can be reconnected again after imaging for additional staining.
- Processing device can also be used for in-situ scanning with adopted fluorescence scanner for special applications.

The invention can be applied very broadly. Any application that requires free immuno-magnetic beads to be separated from a bead/cell mixture may particularly benefit from the invention. Also, any application which requires cells to be magnetically fixed to a surface while reagents are flushed over the cells may use this invention.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A method for the processing of a sample fluid for optical processing, wherein said sample fluid comprises different components (M, C+M) with magnetic particles (M), said method comprising the following steps:
a) introducing the sample fluid into a first compartment (120-620) of a container (110-510);
b) letting in a first phase at least one selected component (M) of the sample pass through a filtering element (140-640) that separates the first compartment (120-620) from a second compartment (130-630) while the passage of at least one other component (C+M) is blocked;
c) collecting the at least one other component (C+M) of the sample on an optical surface (150-650) that is disposed in the first compartment (120-620) or collecting the at least one selected component (M) on an optical surface that is disposed in the second compartment (130-630) of the container; and
d) generating during the first phase a magnetic field (B1) with a nonzero field gradient across the filtering element (140-640) and with field lines that are perpendicular to the filtering element;
the method being **characterized by**:
e) generating after the first phase a magnetic field (B2) with field lines that are parallel to the filtering element (140-640).

2. A processing device (100-600) for processing a sample fluid containing different components (M, C+M), particularly a sample fluid with components comprising magnetic particles (M), comprising:
- a container (110-510) with a first compartment (120-620) that can be filled with the sample fluid and with a second compartment (130-630);
- a filtering element (140-640) that is disposed between said compartments (120-620, 130-630) and that allows in a first phase the passage of at least one selected component (M) of the sample while the passage of at least one other component (C+M) is blocked;
- an optical surface (150-650) that is disposed in the first compartment (120-620) and on which the at least one other component (C+M) of the sample can collect or that is disposed in the second compartment (130-630) and on which the at least one selected component (M) of the sample can collect; and
- a first magnet (160-460) arranged to generate during the first phase a magnetic field (B1) with a nonzero field gradient across the filtering element (140-640) and with field lines running substantially perpendicular to said filtering element;
the processing device being **characterized by** further comprising a further magnet (170-670) arranged to generate after the first phase a magnetic field (B2) that has field lines running substantially in parallel with said filtering element.

3. The method according to claim 1 or the processing device according to claim 2, wherein the magnetic field (B2) generated after the first phase is uniform over the optical surface.

4. The method according to claim 1 or the processing device (100-600) according to claim 2,
**characterized in that** the filtering element (140-640) comprises a structure that is capable of leaving objects below a certain size through while blocking large ones.

5. The method according to claim lor the processing device (100-600) according to claim 2,
**characterized in that** the filtering element (140-640) specifically binds and/or repels at least one component of the sample.

6. The method according to claim 1 or the processing device (100-600) according to claim 2,
**characterized in that** the method or the processing device is adapted to produce a hydrodynamic pressure gradient across the filter element (140-640).

7. The method according to claim 1 or the processing device (100-600) according to claim 2,
**characterized in that** the optical surface (150-650) is detachable from the container (110-510) such that it can be reattached.

8. The method according to claim 1 or the processing device (400) according to claim 2,
**characterized in that** features required for optical analysis such as a microscope objective (490) are arranged at the container (410) for observing the optical surface (450).

9. The method according to claim 1 or the processing device (100-600) according to claim 2,
**characterized in that** the optical surface (150-650) comprises binding sites that can specifically bind at least one component (C+M) of the sample.

10. The method according to claim 1 or the processing device (100-600) according to claim 2,
**characterized in that** the first compartment (120-620) and/or the second compartment (130-630) is provided with at least one fluidic connection (121, 131, 122, 132) for generating a fluid flow through the compartment (120-620).

11. The method according to claim 1 or the processing device (100-600) according to claim 2,
**characterized in that** the first compartment (120-420) and/or the second compartment comprises a single chamber, having on one side the filtering element (140-440) and on another side the optical surface (150-450).

## Patentansprüche

1. Verfahren zum Verarbeiten einer Probenflüssigkeit zur optischen Verarbeitung, wobei die Probenflüssigkeit verschiedene Komponenten (M, C+M) mit magnetischen Teilchen (M) umfasst, wobei das Verfahren die folgenden Schritte umfasst:
a) Einführen der Probenflüssigkeit in eine erste Kammer (120-620) eines Behälters (110-510);
b) Durchlassen, in einer ersten Phase, mindestens einer ausgewählten Komponente (M) der Probe durch ein Filterelement (140-640), das die erste Kammer (120-620) von einer zweiten Kammer (130-630) trennt, während der Durchlass mindestens einer anderen Komponente (C+M) blockiert wird;
c) Sammeln der mindestens einen weiteren Komponente (C+M) der Probe auf einer optischen Oberfläche (150-650), die in der ersten Kammer (120-620) angeordnet ist, oder Sammeln der mindestens einen ausgewählten Komponente (M) auf einer optischen Oberfläche, die in der zweiten Kammer (130-630) des Behälters angeordnet ist; und
d) Erzeugen, während der ersten Phase, eines Magnetfeldes (B1) mit einem Feldgradienten ungleich Null über das Filterelement (140-640) und mit Feldlinien, die senkrecht zu dem Filterelement sind;
wobei das Verfahren durch Folgendes gekennzeichnet ist:
e) Erzeugen, nach der ersten Phase, eines Magnetfeldes (B2) mit zum Filterelement (140-640) parallelen Feldlinien.

2. Verarbeitungsvorrichtung (100-600) zum Verarbeiten einer Probenflüssigkeit, die verschiedene Komponenten (M, C+M) enthält, insbesondere einer Probenflüssigkeit mit Komponenten, die magnetische Teilchen (M) umfassen, umfassend:
- einen Behälter (110-510) mit einer ersten Kammer (120-620), die mit der Probenflüssigkeit gefüllt werden kann, und mit einer zweiten Kammer (130-630);
- ein Filterelement (140-640), das zwischen den Kammern (120-620, 130-630) angeordnet ist und das in einer ersten Phase den Durchlass mindestens einer ausgewählten Komponente (M) der Probe ermöglicht, während der Durchlass mindestens einer anderen Komponente (C+M) blockiert wird;
- eine optische Oberfläche (150-650), die in der ersten Kammer (120-620) angeordnet ist und auf der sich die mindestens eine andere Komponente (C+M) der Probe sammeln kann, oder die in der zweiten Kammer (130-630) angeordnet ist und auf der sich die mindestens eine ausgewählte Komponente (M) der Probe sammeln kann; und
- einen Magneten (160-460), der angeordnet ist, um während der ersten Phase ein Magnetfeld (B1) mit einem Feldgradienten ungleich Null über das Filterelement (140-640) und mit Feldlinien zu erzeugen, die senkrecht zu dem Filterelement sind;
wobei die Verarbeitungsvorrichtung **dadurch gekennzeichnet ist, dass** sie weiterhin einen weiteren Magneten (170-670) umfasst, der angeordnet ist, um nach der ersten Phase ein Magnetfeld (B2) zu erzeugen, dessen Feldlinien im Wesentlichen parallel zu dem Filterelement verlaufen.

3. Verfahren nach Anspruch 1 oder Verarbeitungsvorrichtung nach Anspruch 2, wobei das nach der ersten Phase erzeugte Magnetfeld (B2) über die optische Oberfläche gleichmäßig ist.

4. Verfahren nach Anspruch 1 oder Verarbeitungsvorrichtung (100-600) nach Anspruch 2,
**dadurch gekennzeichnet, dass** das Filterelement (140-640) eine Struktur umfasst, die in der Lage ist, Objekte unterhalb einer bestimmten Größe durchzulassen, während große Objekte blockiert werden.

5. Verfahren nach Anspruch 1 oder Verarbeitungsvorrichtung (100-600) nach Anspruch 2,
**dadurch gekennzeichnet, dass** das Filterelement (140-640) mindestens eine Komponente der Probe spezifisch bindet und/oder abstößt.

6. Verfahren nach Anspruch 1 oder Verarbeitungsvorrichtung (100-600) nach Anspruch 2,
**dadurch gekennzeichnet, dass** das Verfahren oder die Verarbeitungsvorrichtung ausgelegt ist, einen hydrodynamischen Druckgradienten über das Filterelement (140-640) zu erzeugen.

7. Verfahren nach Anspruch 1 oder Verarbeitungsvorrichtung (100-600) nach Anspruch 2,
**dadurch gekennzeichnet, dass** die optische Oberfläche (150-650) vom Behälter (110-510) abnehmbar ist, so dass sie erneut angebracht werden kann.

8. Verfahren nach Anspruch 1 oder Verarbeitungsvorrichtung (400) nach Anspruch 2,
**dadurch gekennzeichnet, dass** für die optische Analyse erforderliche Merkmale wie ein Mikroskopobjektiv (490) am Behälter (410) zur Beobachtung der optischen Oberfläche (450) angeordnet sind.

9. Verfahren nach Anspruch 1 oder Verarbeitungsvorrichtung (100-600) nach Anspruch 2,
**dadurch gekennzeichnet, dass** die optische Oberfläche (150-650) Bindungsstellen umfasst, die an mindestens einer Komponente (C+M) der Probe spezifisch binden können.

10. Verfahren nach Anspruch 1 oder Verarbeitungsvorrichtung (100-600) nach Anspruch 2,
**dadurch gekennzeichnet, dass** die erste Kammer (120-620) und/oder die zweite Kammer (130-630) mit mindestens einer Fluidverbindung (121, 131, 122, 132) zum Erzeugen eines Fluidstroms durch die Kammer (120-620) versehen ist.

11. Verfahren nach Anspruch 1 oder Verarbeitungsvorrichtung (100-600) nach Anspruch 2,
**dadurch gekennzeichnet, dass** die erste Kammer (120-420) und/oder die zweite Kammer eine einzige Kammer umfasst, die auf einer Seite das Filterelement (140-440) und auf einer anderen Seite die optische Oberfläche (150-450) aufweist.

## Revendications

1. Procédé pour le traitement d'un échantillon de fluide pour le traitement optique, dans lequel ledit échantillon de fluide comprend différents constituants (M, C+M) avec des particules magnétiques (M), ledit procédé comprenant les étapes suivantes :
a) l'introduction de l'échantillon de fluide dans un premier compartiment (120-620) d'un récipient (110-510) ;
b) le fait de laisser dans une première phase au moins un constituant sélectionné (M) de l'échantillon passer à travers un élément de filtrage (140-640) qui sépare le premier compartiment (120-620) d'un second compartiment (130-630) pendant que le passage d'au moins un autre constituant (C+M) est bloqué ;
c) la collecte de l'au moins un autre constituant (C+M) de l'échantillon sur une surface optique (150-650) qui est disposée dans le premier compartiment (120-620) ou la collecte de l'au moins un constituant sélectionné (M) sur une surface optique qui est disposée dans le second compartiment (130-630) du récipient ; et
d) la génération, pendant la première phase, d'un champ magnétique (B1) avec un gradient de champ différent de zéro sur l'élément de filtrage (140-640) et avec des lignes de champ qui sont perpendiculaires à l'élément de filtrage ;
le procédé étant **caractérisé par** :
e) la génération, après la première phase, d'un champ magnétique (B2) avec des lignes de champs qui sont parallèles à l'élément de filtrage (140-640).

2. Dispositif de traitement (100-600) pour traiter un échantillon de fluide contenant différents constituants (M, C+M), en particulier un échantillon de fluide avec des constituants comprenant des particules magnétiques (M), comprenant :
- un récipient (110-510) avec un premier compartiment (120-620) qui peut être rempli avec l'échantillon de fluide et avec un second compartiment (130-630) ;
- un élément de filtrage (140-640) qui disposé entre lesdits compartiments (120-620, 130-630) et qui permet dans une première phase le passage d'au moins un constituant sélectionné (M) de l'échantillon pendant que le passage d'au moins un autre constituant (C+M) est bloqué ;
- une surface optique (150-650) qui disposée dans le premier compartiment (120-620) et sur laquelle l'au moins un autre constituant (C+M) de l'échantillon peut s'accumuler ou qui est disposée dans le second compartiment (130-630) et sur laquelle l'au moins un constituant sélectionné (M) de l'échantillon peut s'accumuler ; et
- un premier aimant (160-460) agencé pour générer pendant la première phase un champ magnétique (B1) avec un gradient de champ différent de zéro sur l'élément de filtrage (140-640) et avec des lignes de champ s'étendant sensiblement perpendiculaires audit élément de filtrage ;
le dispositif de traitement étant **caractérisé en ce qu'**il comprend en outre un aimant supplémentaire (170-670) agencé pour générer après la première phase un champ magnétique (B2) qui présente des lignes de champ s'étendant sensiblement en parallèle dudit élément de filtrage.

3. Procédé selon la revendication 1 ou dispositif de traitement selon la revendication 2, dans lequel le champ magnétique (B2) généré après la première phase est uniforme sur la surface optique.

4. Procédé selon la revendication 1 ou dispositif de traitement (100-600) selon la revendication 2,
**caractérisé en ce que** l'élément de filtrage (140-640) comprend une structure qui est apte à laisser passer des objets en dessous d'une certaine taille tout en bloquant les gros.

5. Procédé selon la revendication 1 ou dispositif de traitement (100-600) selon la revendication 2,
**caractérisé en ce que** l'élément de filtrage (140-640) se lie spécifiquement et/ou repousse au moins un constituant de l'échantillon.

6. Procédé selon la revendication 1 ou dispositif de traitement (100-600) selon la revendication 2,
**caractérisé en ce que** le procédé ou le dispositif de traitement est conçu pour produire un gradient de pression hydrodynamique sur l'élément filtre (140-640).

7. Procédé selon la revendication 1 ou dispositif de traitement (100-600) selon la revendication 2,
**caractérisé en ce que** la surface optique (150-650) peut être détachée du récipient (110-510) de sorte qu'elle puisse être rattachée.

8. Procédé selon la revendication 1 ou dispositif de traitement (400) selon la revendication 2,
**caractérisé en ce que** des composants requise pour une analyse optique, par exemple un objectif de microscope (490), sont agencés au niveau du récipient (410) pour observer la surface optique (450).

9. Procédé selon la revendication 1 ou dispositif de traitement (100-600) selon la revendication 2,
**caractérisé en ce que** la surface optique (150-650) comprend des sites de liaisons qui peuvent se lier spécifiquement à au moins un constituant (C+M) de l'échantillon.

10. Procédé selon la revendication 1 ou dispositif de traitement (100-600) selon la revendication 2,
**caractérisé en ce que** le premier compartiment (120-620) et/ou le second compartiment (130-630) est doté d'au moins un raccord fluidique (121, 131, 122, 132) pour générer un écoulement de fluide à travers le compartiment (120-620).

11. Procédé selon la revendication 1 ou dispositif de traitement (100-600) selon la revendication 2,
**caractérisé en ce que** le premier compartiment (120-420) et/ou le second compartiment comprend une chambre unique, présentant sur un côté l'élément de filtrage (140-440) et sur un autre côté la surface optique (150-450).
